# FASCICULE DE BREVET EUROPEEN

(11) **EP 1 244 399 B1**
(45) Date de publication et mention de la délivrance du brevet: **28.05.2008**
(21) Numéro de dépôt: 00993493.6
(22) Date de dépôt: 21.12.2000
(51) Int. Cl.: A61F 5/00

(54) **ANNEAU DE GASTROPLASTIE DESSERRABLE**
LÖSBARER RING ZUR VERFORMUNG DES MAGENS
GASTROPLASTY RING THAT CAN BE LOOSENED

(30) Priorité: 21.12.1999 FR 9916201
(43) Date de publication de la demande: 02.10.2002
(73) Titulaire: Compagnie Européenne d'Etude et de Recherche de Dispositifs pour l'Implantation par Laparoscopie, 38200 Vienne (FR)
(72) Inventeur: BENCHETRIT, Salomon, F-69300 Caluire (FR)
(74) Mandataire: Martin, Didier Roland Valéry
(86) Numéro de dépôt international: PCT/FR2000/003651
(87) Numéro de publication internationale: WO 2001/045597

(56) Documents cités:
- DE-A- 19 751 733
- DE-U- 9 014 048
- US-A- 4 701 163
- US-A- 5 074 868

## Description

### DOMAINE TECHNIQUE

La présente invention se rapporte au domaine technique des implants chirurgicaux destinés à traiter l'obésité par implantation d'une bande gastrique souple destinée à resserrer l'estomac d'un patient, ladite bande gastrique étant pourvue d'une chambre de compression annulaire à volume variable et réglable par l'intermédiaire d'un cathéter de réglage relié à un dispositif de réglage et de commande implanté dans le corps du patient.

La présente invention concerne un anneau de gastroplastie formé par une bande souple destinée à être fermée autour de l'estomac sensiblement vers et par ses deux extrémités, à l'aide d'un système de fermeture pour réduire le diamètre de l'ouverture de stoma, ladite bande comportant une chambre de compression annulaire à volume réglable, reliée par un cathéter de réglage à un dispositif de réglage de la pression interne de ladite chambre, de manière à régler son expansion diamétrale.

### TECHNIQUE ANTERIEURE

Dans le cas de patients atteints d'obésité extrêmement sévère (obésité morbide), c'est-à-dire dans le cas de patients dont le poids excède le poids idéal d'au moins cinquante kilos par exemple, il est absolument nécessaire d'intervenir de manière chirurgicale sur de tels patients afin d'éviter non seulement une série de problèmes de santé découlant de cette obésité, mais encore pour éviter une mort certaine et proche de tels patients.

En effet, il est acquis que les patients souffrant d'obésité morbide voient leur espérance de vie réduite de manière importante et d'au moins une dizaine à une quinzaine d'années, tout en créant d'importants problèmes de charges psychologiques. Par ailleurs, toute une série de phénomènes annexes de santé sont impliqués, ayant une incidence sur l'apparition de maladies cardio-vasculaires, d'hypertension, de diabète, d'arthrites sévères notamment.

Il est également apparu que des traitements basés sur une diète sévère combinée avec une série d'exercices physiques associés à une modification du comportement, notamment alimentaire, étaient peu adaptés à de tels cas d'obésité morbide, bien que de telles méthodes de traitement soient les plus saines.

C'est la raison pour laquelle les traitements efficaces et à long terme de traitement de l'obésité morbide font intervenir un traitement chirurgical.

De manière générale, on distingue les techniques de traitement chirurgical faisant intervenir un défaut d'absorption des aliments, c'est-à-dire un raccourcissement du passage classique de l'aliment et des sucs digestifs et les techniques faisant intervenir une restriction gastrique, réduisant la taille de l'estomac.

Les techniques chirurgicales impliquant un défaut d'absorption sont par exemple celles impliquant une technique de by-pass ou dérivation du petit intestin ou encore celles mettant en oeuvre une séparation du passage des aliments relativement aux sucs digestifs. La technique chirurgicale de by-pass peut donner lieu à de sévères complications, de telle sorte que cette technique n'est plus que très rarement utilisée. La technique chirurgicale de séparation de passage du bol alimentaire relativement aux sucs digestifs ne fait pas intervenir de complications particulières, mais nécessite une intervention chirurgicale importante impliquant notamment une gastrectomie partielle.

C'est la raison pour laquelle on tend désormais à utiliser les techniques chirurgicales mettant en oeuvre une restriction gastrique pour réduire la prise d'aliments.

De telles techniques font intervenir de manière classique l'utilisation d'anneaux de gastroplastie implantés autour de l'estomac pour réduire sa taille et le diamètre de son passage (stoma).

La plupart des dispositifs de gastroplastie connus, et par exemple celui décrit dans le brevet US-A-5074868, mettent en oeuvre une bande souple réalisée en matériau élastomère et destiné à être implanté autour de l'estomac puis resserré et fermé suivant une boucle de diamètre fixe par un système de fermeture. Le corps de la bande souple comporte une cavité ou chambre de compression à volume variable reliée par un cathéter de réglage à un dispositif de réglage de la pression interne de la chambre afin de faire varier le diamètre interne de la boucle pour modifier ou régler le diamètre du stoma par injection ou extraction d'un volume de liquide hors de la chambre. Une telle opération de réglage du diamètre interne de l'anneau s'effectue à l'aide de dispositifs classiques de commande incluant un boîtier miniaturisé implanté directement sous la peau du patient et pourvu d'une membrane auto-obturante à travers laquelle le médecin injecte ou retire du liquide par une seringue.

Le modèle d'utilité DE-G-90 14048 présente un système partiellement annulaire spécifiquement conçu et adapté à la fermeture totale de vaisseaux sanguins, dont l'enseignement n'est en outre pas transposable à la compression de l'estomac qui doit être parfaitement maîtrisée.

Le système de fermeture du brevet US-A-5074868 met en oeuvre un suturage, à l'aide de fils de suture, des deux brins de la bande souple de l'anneau.

Un tel dispositif donne généralement satisfaction, mais souffre comme la plupart des systèmes connus, d'inconvénients liés essentiellement à la difficulté de toute intervention chirurgicale susceptible de survenir après la pose de l'implant de gastroplastie. En effet, il s'avère que malgré la possibilité de modifier dans une certaine mesure le diamètre de l'anneau sans intervention chirurgicale par le boîtier miniaturisé mentionné ci-dessus, la pose de tels implants gastriques peut s'accompagner de phénomènes d'intolérance, par exemple accompagné de vomissements, liés à une trop forte réduction du diamètre de stoma, ou encore à une action inefficace de l'implant lié à un diamètre du stoma trop important, ou encore tout simplement à une gêne ou une infection ou inflammation locale ou générale.

C'est la raison pour laquelle il est souvent nécessaire d'intervenir à nouveau de manière chirurgicale, soit pour soulager le patient, soit pour modifier ou changer l'anneau de gastroplastie préalablement implanté. De telles interventions chirurgicales sont particulièrement sévères et nécessitent de plus, soit la découpe de l'anneau par un chirurgien, soit comme dans le cas du brevet US-A-5074868 la coupe du fil de suture s'accompagnant d'une ouverture complète de l'anneau puis son changement et son remplacement.

En définitive, de telles opérations sont délicates à réaliser, difficilement supportées par le patient, coûteuses, et ce d'autant plus qu'elles impliquent la destruction d'un implant et son remplacement. En outre, dans le cas du brevet US-A-5074868, le dispositif d'aide à la coupe de la suture rend l'implant chirurgical relativement complexe à fabriquer et réaliser, sans pour autant constituer une aide essentielle lors de l'opération.

### EXPOSE DE L'INVENTION

L'objet assigné à l'invention vise en conséquence à proposer un nouvel anneau de gastroplastie permettant de porter remède aux différents inconvénients énumérés précédemment et susceptible de faciliter d'éventuelles ré interventions chirurgicales après la pose de l'implant, ne nécessitant pas le remplacement de l'implant, ce dernier étant dans tous les cas, de conception particulièrement simple et facile à réaliser.

Un autre objet de l'invention vise à proposer un nouvel anneau de gastroplastie susceptible d'assurer de manière simple et fiable une fermeture réversible de la boucle constituant l'anneau.

Un autre objet de l'invention vise à proposer un nouvel anneau de gastroplastie susceptible de proposer des moyens simples et fiables d'adaptation du diamètre de l'anneau à chaque situation chirurgicale donnée.

Un autre objet de l'invention vise à proposer un nouvel anneau de gastroplastie susceptible de présenter plusieurs diamètres d'implantation.

Un autre objet de l'invention vise à proposer un nouvel anneau de gastroplastie permettant de réduire la gêne ressentie par le patient tout en étant fermement maintenu en place par la boucle.

Un autre objet de l'invention vise à proposer un nouvel anneau de gastroplastie particulièrement facile à fabriquer tout en étant d'une excellente résistance mécanique générale.

Les objets assignés à l'invention sont atteints à l'aide d'un anneau de gastroplastie formé par une bande souple destinée à être fermée vers ses deux extrémités par un système de fermeture autour de l'estomac pour réduire le diamètre de l'ouverture du stoma, ladite bande comportant une chambre de compression annulaire à volume réglable et à section sensiblement constante reliée par un cathéter de réglage à un dispositif de réglage de la pression interne de ladite chambre, pour régler son expansion diamétrale, caractérisé en ce que le système de fermeture comporte des moyens de blocage et desserrage réversibles de l'anneau, comprenant un moyen mâle et un moyen femelle formant une cavité dans ou contre laquelle vient s'expanser et se bloquer de manière réversible le moyen mâle, permettant d'assurer, à partir de sa position de blocage diamétral, son relâchement diamétral momentané par déplacement relatif des deux extrémités, tout en formant constamment une boucle fermée autour de l'estomac.

### DESCRIPTIF SOMMAIRE DES DESSINS

D'autres objets et avantages de l'invention apparaîtront mieux à la lecture de la description ci-jointe, ainsi qu'à l'aide des dessins annexés, à titre purement illustratif et informatif dans lesquels :
- La figure 1 illustre selon une vue schématique en perspective, un exemple de réalisation d'un anneau de gastroplastie conforme à l'invention en position de fermeture.
- La figure 2 illustre selon une vue schématique en perspective, un exemple de réalisation d'un anneau de gastroplastie conforme à l'invention en position d'ouverture avant son implantation.
- La figure 3 illustre selon une vue partielle en coupe transversale un détail de la section d'un anneau de gastroplastie conforme à l'invention.
- La figure 4 illustre selon une vue en perspective une variante de réalisation d'un anneau de gastroplastie conforme à l'invention.
- La figure 5 illustre selon une vue en coupe partielle un détail de réalisation d'une variante d'un anneau de gastroplastie conforme à l'invention.

### MEILLEURE MANIERE DE REALISER L'INVENTION

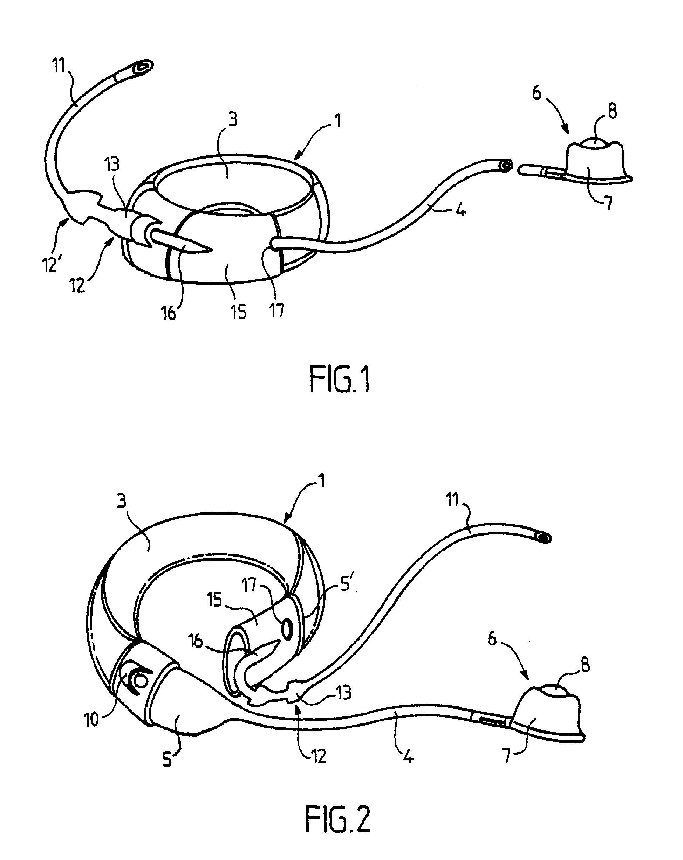

Les figures 1 et 2 illustrent un exemple de réalisation préférentielle d'un anneau de gastroplastie conforme à l'invention, formé par une bande souple 1 réalisée par exemple par thermoformage à partir d'un matériau élastomère à usage chirurgical, ladite bande 1 définissant, de préférence sensiblement sur toute sa longueur, une chambre de compression interne 2 à section sensiblement constante, délimitée par les parois 3 de la bande souple 1 et par deux zones d'extrémités 5, 5'. Dans sa position d'implantation dans l'estomac d'un patient, tel qu'illustré à la figure 1, la chambre de compression 2 forme donc une chambre de compression annulaire dont la section et le profil sont adaptés pour éviter la formation d'une zone de pincement susceptible de générer des plis avec les parois de l'estomac. Ceci implique notamment une surface de contact continue et uniforme avec l'estomac et la formation d'une zone de compression annulaire presque parfaite excluant notamment une forme en gouttelette, susceptible d'agresser les tissus cellulaires.

Tel que cela est bien connu dans l'art antérieur, la chambre de compression 2 définit un volume fermé et interne à l'anneau de gastroplastie, qui est destiné à former un volume réglable de manière à régler l'expansion diamétrale de l'anneau lorsqu'il est en place pour l'adapter à chaque situation chirurgicale donnée.

De manière classique, le réglage de l'expansion diamétrale de l'anneau de gastroplastie conforme à l'invention est assuré par un cathéter de réglage 4 formé par un élément tubulaire en matériau élastomère prolongeant l'extrémité libre 5 de la chambre de compression 2 pour relier ladite chambre à un dispositif de réglage 6 de la pression interne de ladite chambre.

Tel que cela est bien connu de l'homme de l'art, le dispositif de réglage 6 peut être formé par un boîtier miniaturisé 7 implanté sous la peau du patient. Le boîtier miniaturisé 7 comporte par exemple une membrane supérieure 8 auto-obturante destinée à être percée par une seringue pour injecter ou retirer une certaine quantité de fluide (eau distillée par exemple) servant à assurer la variation de volume de la chambre de compression 2, pour régler le volume de la chambre et obtenir ainsi le diamètre interne de l'anneau souhaité. Un tel dispositif étant bien connu de l'homme du métier, il ne sera en conséquence pas décrit plus en détail.

L'anneau de gastroplastie conforme à l'invention comporte également un système de fermeture destiné à fermer et maintenir en position de boucle l'anneau de gastroplastie autour de l'estomac.

Selon une caractéristique importante de l'invention, le système de fermeture conforme à l'invention comporte des moyens de blocage et desserrage réversibles de l'anneau permettant d'assurer, à partir de la position de blocage diamétral de l'anneau correspondant à la position fermée et en boucle tel qu'illustré à la figure 1, son relâchement diamétral momentané par déplacement relatif des deux extrémités de l'anneau, tout en formant constamment une boucle fermée autour de l'estomac.

La réalisation d'une telle fonction technique permet de réduire la gravité et l'importance d'éventuelles ré interventions chirurgicales suivant la pose d'un implant en évitant d'avoir à sectionner et détruire l'anneau de gastroplastie mis en place, une telle fonction permettant de laisser l'anneau en place et d'assurer une simple augmentation momentanée de son diamètre sans détruire la boucle de l'anneau, ce qui permet un resserrage ultérieur pour revenir à la position de fermeture illustré à la figure 1.

Selon une variante préférentielle de l'invention, tel qu'illustré aux figures 1 à 3, l'anneau de gastroplastie conforme à l'invention comprend un système de fermeture qui comporte :
- un moyen mâle et un moyen femelle solidaires de la bande 1, et par exemple situés sensiblement vers ses deux extrémités 5, 5', le moyen mâle et le moyen femelle étant montés sur la bande, c'est-à-dire la prolongeant à ces deux extrémités 5, 5', de telle façon que lors de leur connexion mutuelle, la bande 1 est fermée en formant une boucle,
- les moyens de blocage et desserrage réversibles étant montés sur le moyen mâle et susceptibles de coulisser dans le moyen femelle 10 entre une position de blocage et au moins une position de desserrage, tout en permettant le maintien d'une boucle.

La variante de réalisation préférentielle illustrée aux figures montre que le moyen femelle est formé par un oeillet 10 ménagé à la périphérie de la bande 1 dans une position adjacente à l'extrémité 5. Le moyen mâle est formé par un cathéter gonflable 11 réalisé en un matériau élastomère biocompatible, destiné à être enfilé dans l'oeillet 10 dans la position de fermeture de l'anneau et à servir en outre de moyen de guidage.

Les moyens de blocage et desserrage réversibles sont reliés au cathéter de gonflage 11 et comportent une zone déformable 12 susceptible de former une excroissance 13 en cas d'augmentation de pression dans le cathéter de gonflage 11, ladite excroissance 13 prenant appui dans ou contre le moyen femelle 10 pour bloquer l'anneau en position de fermeture. L'excroissance 13 reprend sa forme de repos en cas de retour à la pression normale dans le cathéter de gonflage 11 pour permettre le coulissement et le guidage libre dudit cathéter dans l'oeillet 10 et le desserrage de la boucle.

Les moyens de blocage et desserrage réversibles sont avantageusement formés sur le cathéter de gonflage 11 (figures 1, 2, 5) et sont destinés à venir coopérer avec l'oeillet 10 pour assumer, lorsque l'oeillet 10 est connecté avec le cathéter de gonflage 11, le blocage et le desserrage réversibles de l'anneau.

Selon une version particulièrement avantageuse de l'invention, la zone déformable réversible 12 est formée par au moins une zone 13 de moindre résistance susceptible d'une part, de former localement une excroissance en cas d'augmentation de pression dans le cathéter de gonflage 11, ladite excroissance prenant alors appui dans ou contre l'oeillet 10 pour bloquer l'anneau en position de fermeture, et d'autre part étant susceptible de reprendre sa forme de repos correspondant alors sensiblement au diamètre normal du cathéter 11 en cas de retour à la pression normale, pour permettre le coulissement libre du cathéter gonflable 11 dans l'oeillet 10, ce qui induit le desserrage de la boucle. La zone 13 de moindre résistance peut s'étendre sur toute la section périphérique du cathéter 11 pour former une boulle, ou sur une fraction seulement pour former alors un simple dôme en extension.

Avantageusement, la zone déformable 12 peut être formée par une section du cathéter de gonflage 11 présentant une dureté du matériau élastomère composant le cathéter gonflable 11, qui est localement inférieure à la dureté générale du cathéter de gonflage 11. Dans un tel cas, le cathéter de gonflage 11 étant relié à un dispositif externe de mise en pression par l'intermédiaire d'un fluide (air ou liquide), la zone 12 tendra à former un ballonnet 13 d'un diamètre supérieur au diamètre de l'oeillet 10, ce qui assure le blocage diamétral de l'anneau.

Bien évidemment, à titre de variante, un anneau de gastroplastie conforme à l'invention pourra comprendre plusieurs zones 12 de moindre résistance, espacées de manière régulière ou non le long du cathéter de gonflage 11 pour constituer un anneau de gastroplastie susceptible de comporter plusieurs diamètres fixes de pose. Avantageusement, le cathéter de gonflage 11 comporte deux zones 12, 12' de moindre résistance.

Selon cette réalisation préférentielle, les moyens de blocage et de desserrage font intervenir des moyens de fluide (pneumatiques ou hydrauliques, air, fluide, ou liquide, par exemple) assurant la fonction de blocage et desserrage réversibles.

Avantageusement, la bande souple 1 est pourvue à une extrémité, et par exemple à l'extrémité 5' opposée à l'extrémité 5, d'un manchon creux 15 prolongeant la bande souple 1, manchon creux 15 sur lequel est fixée une extrémité 16 du cathéter gonflable 11. Le manchon creux 15 comporte également une ouverture 17 ménagée dans l'une de ses faces, de préférence une face externe, de telle manière que l'autre extrémité 5 de la bande souple 1 puisse s'insérer dans ledit manchon en position de fermeture, le cathéter de réglage 11 passant alors dans l'ouverture 17 pour former la boucle de l'anneau (figure 2). Une telle disposition constructive assure une bonne fiabilité de la tenue de la fermeture, tout en permettant à la chambre de compression 2 de s'étendre sur tout le périmètre de serrage de l'estomac.

Tel que cela a déjà été décrit précédemment, l'oeillet 10 est avantageusement disposé à distance de l'extrémité 5 de la bande pour créer une portion libre de la chambre de compression 2 destinée à venir s'insérer dans le manchon creux 15 en vue de la mise en place de l'anneau dans sa position de boucle.

Avantageusement, la bande souple 1, la chambre de compression 2 ainsi que le moyen femelle 10 forment une pièce monobloc réalisée à partir d'un même matériau plastique élastomère, le cathéter de réglage 4 et le cathéter de gonflage 11, indépendants entre eux, étant ensuite thermosoudés.

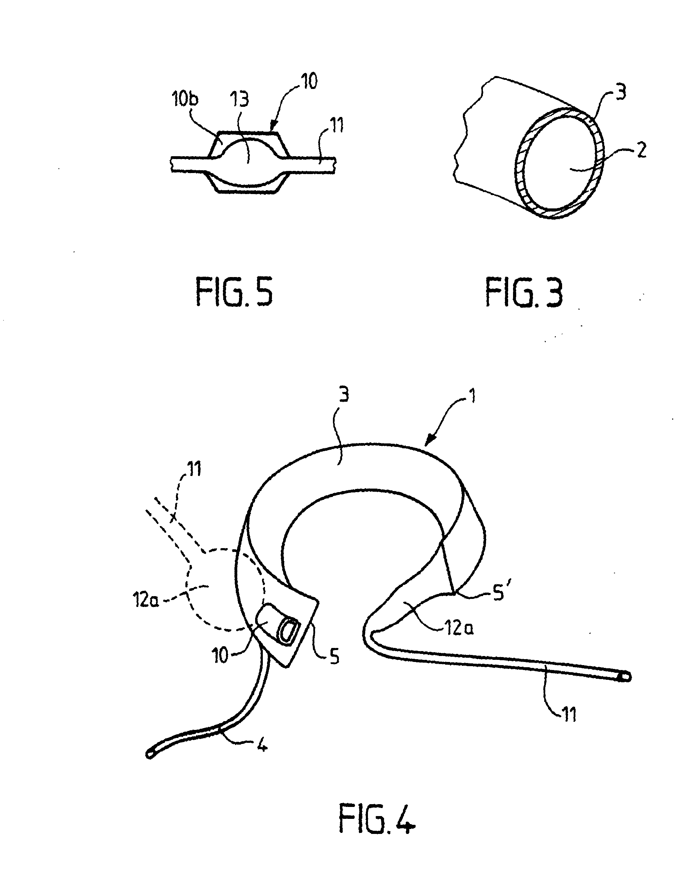

La figure 5 illustre une variante de réalisation qui ne diffère de celle des figures 1 à 3 que par la configuration géométrique du moyen femelle 10 en vue d'améliorer le blocage. Selon cette variante, l'oeillet 10 comporte ou est formé par une cavité traversante 10b dans laquelle l'excroissance 13, ou le ballonnet, vient s'expanser et se bloquer en position de manière réversible, selon la pression du fluide. La cavité 10b peut être située dans la continuité de la section annulaire de la chambre 2 pour former un anneau régulier et être formée en totalité ou en partie par le manchon creux 15 ou dans son prolongement sur l'autre extrémité de l'anneau.

La figure 4 illustre une autre variante de réalisation, qui ne diffère des variantes précédentes que par la configuration de la zone déformable 12. Cette dernière est formée par une poche 12a sensiblement oblongue, solidaire de la bande souple 1, et par exemple prolongeant l'extrémité 5' et fixée à cette dernière. Le cathéter de gonflage 11 prolonge la poche 12a et est branché à son extrémité terminale. Lors de la fermeture de l'anneau, le cathéter 11 puis la poche 12a sont enfilés dans l'oeillet 10, la poche 12a (ou la majorité de sa longueur) dépassant hors de l'oeillet 10. La mise en pression du cathéter de gonflage 11 provoque le gonflement de la poche 12a tel qu'indiqué en pointillé sur la figure 4, causant le blocage et la fermeture de l'anneau.

La réalisation d'un anneau de gastroplastie monobloc permet de simplifier le procédé de fabrication de l'anneau et d'obtenir un anneau ne présentant pas de risque d'altération dans le temps.

Lors de l'implantation, l'anneau conforme à l'invention est mis en place autour de l'estomac dans la positon illustrée à la figure 2. Le dispositif de réglage 6 étant au préalable déconnecté, le chirurgien assure le passage du cathéter de réglage 4 dans l'ouverture 17 pour insérer l'extrémité 5 dans le manchon creux 15 (figure 1). Le chirurgien peut ensuite, par l'intermédiaire d'un dispositif de gonflage (non représenté aux figures) raccordé au cathéter de gonflage 11, assurer le blocage en position de l'anneau, en ayant au préalable veillé à positionner la zone de moindre résistance 12 correspondant au diamètre de l'implant souhaité, en regard de l'oeillet 10. Le chirurgien peut ensuite assurer le réglage du diamètre interne de l'anneau en injectant ou retirant la quantité de liquide idoine par l'intermédiaire du dispositif de réglage 6.

En cas de ré opération chirurgicale, il est possible grâce à l'anneau de gastroplastie conforme à l'invention, de se limiter à réaliser, par coelioscopie ou laparoscopie, un examen superficiel extérieur de la situation de l'implant, par simple inspection optique à l'aide d'une caméra. Eventuellement, si la situation le nécessite, il est possible dans un premier temps, par simple coelioscopie de mettre en dépression le cathéter de gonflage 11, ce qui conduit à un relâchement du ballonnet 13 et à un coulissement du cathéter de gonflage 11 à travers l'oeillet 10 en raison de la longueur suffisante dudit cathéter. Un tel coulissement s'accompagne d'un desserrage partiel et momentané de l'anneau, sans avoir à opérer le patient de manière sévère. Il est ensuite possible, également par simple examen et intervention laparoscopique, de renfermer et bloquer l'anneau en position de fermeture de manière très simple, puisque la boucle de l'anneau n'a jamais été détruite.

### POSSIBILITE D'APPLICATION INDUSTRIELLE

L'invention trouve son application industrielle dans la réalisation et l'utilisation d'anneaux de gastroplastie.

## Revendications

1. - Anneau de gastroplastie formé par une bande souple (1) destinée à être fermée vers ses deux extrémités (5, 5') par un système de fermeture (10, 10b, 11, 12, 12', 13) autour de l'estomac pour réduire le diamètre de l'ouverture du stoma, ladite bande comportant une chambre de compression (2) annulaire à volume réglable et à section sensiblement constante reliée par un cathéter de réglage (4) à un dispositif de réglage (6) de la pression interne de ladite chambre, pour régler son expansion diamétrale, **caractérisé en ce que** le système de fermeture (10, 10b, 11, 12, 12', 13) comporte des moyens de blocage et desserrage (10, 10b, 12, 12', 13) réversibles de l'anneau, comportant un moyen mâle (11) et un moyen femelle (10, 10b) formant une cavité dans ou contre laquelle vient s'expanser et se bloquer de manière réversible le moyen mâle (11), permettant d'assurer, à partir de sa position de blocage diamétral, son relâchement diamétral momentané par déplacement relatif des deux extrémités (5, 5'), tout en formant constamment une boucle fermée autour de l'estomac.

2. - Anneau selon la revendication 1 **caractérisé en ce que** les moyens de blocage et de desserrage font intervenir des moyens de fluide, tels que l'air ou un liquide.

3. - Anneau selon la revendication 1 ou 2 **caractérisé en ce que** dans le système de fermeture (10-13) :
- le moyen mâle (11) et le moyen femelle (10) sont solidaires de la bande (1) et montés sur elle en la prolongeant à ses deux extrémités (5, 5') de telle façon que lors de leur connexion mutuelle, la bande (1) est fermée en formant une boucle,
- les moyens de blocage et desserrage (12, 12', 13) réversibles sont montés sur le moyen mâle (11) et susceptibles de coulisser dans le moyen femelle (10) entre une position de blocage et une position de desserrage.

4. - Anneau selon les revendications 2 et 3 **caractérisé en ce que** le moyen femelle est formé par un oeillet (10, 10b), le moyen mâle est formé par un cathéter gonflable (11) destiné à être enfilé dans l'oeillet (10, 10b), les moyens de blocage et desserrage réversibles étant reliés au cathéter (11) et comportant au moins une zone déformable (12, 12') susceptible d'une part de former localement une excroissance (13) en cas d'augmentation de pression dans le cathéter, ladite excroissance prenant appui dans ou contre l'oeillet (10) pour bloquer l'anneau en position de fermeture, et d'autre part de reprendre sa forme de repos en cas de retour à la pression normale, pour permettre le coulissement libre du cathéter gonflable (11) dans l'oeillet (10, 10b) et le desserrage de la boucle.

5. - Anneau selon la revendication 4 **caractérisé en ce que** la zone déformable (12, 12') est une zone de moindre résistance formée sur le cathéter de gonflage (11).

6. - Anneau selon la revendication 5 **caractérisé en ce que** la zone déformable (12) est formée par une poche (12a) prolongeant l'extrémité (5') de la bande souple (1) et sur laquelle le cathéter de gonflage (11) est branché.

7. - Anneau selon la revendication 4 ou 5 **caractérisé en ce que** le cathéter gonflable (11) comporte deux zones (12, 12') de moindre résistance, espacées le long dudit cathéter.

8. - Anneau selon la revendication 4, 5 ou 7 **caractérisé en ce que** la bande souple (1) est pourvue à une extrémité (5') d'un manchon creux (15) sur lequel est fixée une extrémité (16) du cathéter gonflable (11), ledit manchon comportant une ouverture (17) de manière à ce que l'autre extrémité (5) de la bande (1) puisse s'insérer dans le manchon (15), le cathéter de réglage (4) passant dans l'ouverture (17) pour former la boucle de l'anneau.

9. - Anneau selon la revendication 8 **caractérisé en ce que** l'oeillet (10) est disposé à distance d'une extrémité (15) de la bande (1), pour créer une portion libre de la chambre (2) destinée à venir s'insérer dans le manchon creux.

10. -Anneau selon l'une des revendications 1 à 9 **caractérisé en ce que** la bande souple (1), la chambre de compression (2) et le moyen femelle (10, 10b) forment une pièce monobloc réalisée à partir d'un même matériau plastique.

11. -Anneau selon l'une des revendications 1 à 10 **caractérisé en ce que** la chambre de compression (2) s'étend sur la totalité de la périphérie de l'anneau.

12. -Anneau selon l'une des revendications 4 à 11 **caractérisé en ce que** le cathéter gonflable (11) est relié à un dispositif de mise en compression /décompression.

## Claims

1. Gastroplasty ring formed by a flexible band (1) intended to be closed towards its two ends (5, 5') by a closure system (10, 10b, 11, 12, 12', 13) around the stomach in order to reduce the diameter of the stoma, the said band comprising an annular compression chamber (2) with an adjustable volume and a substantially constant cross-section connected by an adjustment catheter (4) to a device (6) for adjusting the internal pressure of the said chamber, in order to adjust its diametral expansion, **characterised in that** the closure system (10, 10b, 11, 12, 12', 13) comprises reversible means (10, 10b, 11, 12, 12', 13) of locking and releasing the ring, comprising a male means (11) and a female means (10, 10b) forming a cavity in or against which the male means (11) is expanded and locked in a reversible manner, making it possible to ensure, from its diametral locking position, its momentary diametral release by relative movement of the two ends (5, 5') whilst constantly forming a closed loop around the stomach.

2. Ring according to claim 1, **characterised in that** the locking and releasing means involve fluid means, such as air or a liquid.

3. Ring according to claim 1 or 2, **characterised in that**, in the closure system (10-13):
- the male means (11) and the female means (10) are fixed to the band (1) and mounted on it while extending it at its two ends (5, 5') so that, when they are mutually connected, the band (1) is closed, forming a loop,
- the reversible locking and release means (12, 12', 13) are mounted on the male means (11) and able to slide in the female means (10) between a locking position and a release position.

4. Ring according to claims 2 and 3, **characterised in that** the female means is formed by an eyelet (10, 10b), the male means is formed by an inflatable catheter (11) intended to be slipped into the eyelet (10, 10b), the reversible locking and release means being connected to the catheter (11) and comprising at least one deformable zone (12, 12') able firstly to form locally a protrusion (13) in the event of increase in pressure in the catheter, the said protrusion bearing in or against the eyelet (10) in order to lock the ring in the closed position, and secondly to resume its idle shape in the event of return to normal pressure, to allow the free sliding of the inflatable catheter (11) in the eyelet (10, 10b) and the release of the loop.

5. Ring according to claim 4, **characterised in that** the deformable zone (12, 12') is a zone of less strength formed on the inflation catheter (11).

6. Ring according to claim 5, **characterised in that** the deformable zone (12, 12') is formed by a pouch (12a) extending the end (5') of the flexible band (1) and to which the inflation catheter (11) is connected.

7. Ring according to claim 4 or 5, **characterised in that** the inflatable catheter (11) comprises two zones (12, 12') of less strength, spaced along the said catheter.

8. Ring according to claim 4, 5 or 7, **characterised in that** the flexible band (1) is provided at one end (5') with a hollow sleeve (15) to which one end (16) of the inflatable catheter (11) is fixed, the said sleeve comprising an opening (17) so that the other end (5) of the band (1) can be inserted in the sleeve (15), the adjustment catheter (4) passing through the opening (17) in order to form the loop of the ring.

9. Ring according to claim 8, **characterised in that** the eyelet 10) is disposed at a distance from one end (15) of the band (1) in order to create a free portion of the chamber (2) intended to be inserted in the hollow sleeve.

10. Ring according to one of claims 1 to 9, **characterised in that** the flexible band (1), the compression chamber (2) and the female means (10, 10b) form a single piece produced from the same plastics material.

11. Ring according to one of claims 1 to 10, **characterised in that** the compression chamber (2) extends over the whole of the periphery of the ring.

12. Ring according to one of claims 4 to 11, **characterised in that** the inflatable catheter (11) is connected to a compressing/decompressing device.

## Patentansprüche

1. - Gastroplastikring, gebildet aus einem elastischen Band (1), dazu vorgesehen, in Richtung auf seine zwei Enden (5, 5') durch ein Verschlusssystem (10, 10b, 11, 12, 12', 13) um den Magen geschlossen zu sein, um den Durchmesser der Öffnung des Stomas zu reduzieren, wobei das Band eine ringförmige Druckkammer (2) mit regulierbarem Volumen und mit einem im Wesentlichen konstanten Querschnitt aufweist, verbunden durch einen Regelkatheter (4) mit einer Vorrichtung zur Regelung (6) des inneren Drucks der besagten Kammer, um seine diametrische Ausdehnung zu regeln, **dadurch gekennzeichnet, dass** das Verschlusssystem (10, 10b, 11, 12, 12', 13) reversible Mittel zum Blockieren und zum Lösen (10, 10b, 12, 12', 13) des Ringes umfasst, die ein Steckmittel (11) und ein Aufnahmemittel (10, 10b) umfassen, das einen Hohlraum bildet, in dem oder gegen welchen das Steckmittel (11) auf reversible Weise ausgedehnt und gesperrt wird, wobei es ermöglicht, ausgehend von seiner diametrischen Blockierstellung durch relative Verschiebung der zwei Enden (5, 5') seine augenblickliche diametrische Lockerung sicherzustellen und gleichzeitig ständig eine geschlossene Schleife um den Magen zu bilden.

2. - Ring nach Anspruch 1, **dadurch gekennzeichnet, dass** die Mittel zum Blockieren und Lösen Fluidmittel einsetzen, wie z.B. Luft oder eine Flüssigkeit.

3. - Ring nach Anspruch 1 oder 2, **dadurch gekennzeichnet, dass** im Verschlusssystem (10-13):
- das Steckmittel (11) und das Aufnahmemittel (10) mit dem Band (1) fest verbunden und auf ihm angebracht sind, indem sie es an seinen zwei Enden (5, 5') so verlängern, dass bei ihrer gegenseitigen Verbindung das Band (1) geschlossen wird, indem es eine Schleife bildet,
- die Mittel zum reversiblen Blockieren und Lösen (12, 12', 13) auf dem Steckmittel (11) angebracht und dazu in der Lage sind, in dem Aufnahmemittel (10) zwischen einer Position des Blockierens und einer Position des Lösens zu gleiten.

4. - Ring nach Anspruch 2 und 3, **dadurch gekennzeichnet, dass** das Aufnahmemittel durch eine Öse (10, 10b) gebildet ist, dass das Steckmittel durch einen aufblasbaren Katheter (11) gebildet ist, der dazu vorgesehen ist, in die Öse (10, 10b) eingefädelt zu werden, wobei die reversiblen Mittel zum Blockieren und Lösen mit dem Katheter (11) verbunden sind und mindestens einen verformbaren Bereich (12, 12') umfassen, der dazu in der Lage ist, einerseits im Fall einer Erhöhung des Drucks im Katheter lokal eine Ausstülpung (13) zu bilden, wobei sich die Ausstülpung in oder gegen die Öse (10) stützt, um den Ring in der Verschlussposition zu blockieren, und andererseits seine Ruheform für den Fall wieder einzunehmen, dass er zum normalen Druck zurückkehrt, um das freie Gleiten des aufblasbaren Katheters (11) in der Öse (10, 10b) und das Lösen der Schleife zu ermöglichen.

5. - Ring nach Anspruch 4, **dadurch gekennzeichnet, dass** der verformbare Bereich (12, 12') ein Bereich geringeren Widerstandes, ausgebildet auf dem Aufblaskatheter (11), ist.

6. - Ring nach Anspruch 5, **dadurch gekennzeichnet, dass** der verformbare Bereich (12) durch eine Tasche (12a) gebildet ist, die das Ende (5') des flexiblen Bandes (1) verlängert, und an den der Aufblaskatheter (11) angeschlossen ist.

7. - Ring nach Anspruch 4 oder 5, **dadurch gekennzeichnet, dass** der aufblasbare Katheter (11) zwei Bereiche (12, 12') geringeren Widerstandes umfasst, die entlang des Katheters beabstandet angeordnet sind.

8. - Ring nach Anspruch 4, 5 oder 7, **dadurch gekennzeichnet, dass** das flexible Band (1) an einem Ende (5') mit einer hohlen Muffe (15) versehen ist, an der ein Ende (16) des aufblasbaren Katheters (11) befestigt ist, wobei die Muffe eine Öffnung (17) umfasst, sodass das andere Ende (5) des Bandes (1) in die Muffe (15) eingeführt werden kann, wobei der Regelkatheter (4) in die Öffnung (17) führt, um die Schleife des Ringes zu bilden.

9. - Ring nach Anspruch 8, **dadurch gekennzeichnet, dass** die Öse (10) im Abstand von einem Ende (15) des Bandes (1) angeordnet ist, um einen freien Abschnitt der Kammer (2) zu bilden, der dazu vorgesehen ist, in die hohle Muffe eingefügt zu werden.

10. - Ring nach einem der Ansprüche 1 bis 9, **dadurch gekennzeichnet, dass** das flexible Band (1), die Druckkammer (2) und das Steckmittel (10, 10b) ein Stück bilden, das aus demselben Kunststoff hergestellt ist.

11. - Ring nach einem der Ansprüche 1 bis 10, **dadurch gekennzeichnet, dass** sich die Druckkammer (2) über den gesamten Umfang des Ringes erstreckt.

12. - Ring nach einem der Ansprüche 4 bis 11, **dadurch gekennzeichnet, dass** der aufblasbare Katheter (11) mit einer Vorrichtung zur Komprimierung /Dekomprimierung verbunden ist.
